(19) Europäisches Patentamt
European Patent Office
Office européen des brevets

(11) Veröffentlichungsnummer: **0 599 892 B1**

(12) # EUROPÄISCHE PATENTSCHRIFT

(45) Veröffentlichungstag der Patentschrift: **22.11.95**

(51) Int. Cl.⁶: **C07C 323/47**, A01N 35/10, A01N 31/06, C07D 261/08, C07D 333/16, C07D 333/28, A01N 43/10, A01N 43/80

(21) Anmeldenummer: **92916831.8**

(22) Anmeldetag: **05.08.92**

(86) Internationale Anmeldenummer:
**PCT/EP92/01774**

(87) Internationale Veröffentlichungsnummer:
**WO 93/03010 (18.02.93 93/05)**

Die Akte enthält technische Angaben, die nach dem Eingang der Anmeldung eingereicht wurden und die nicht in dieser Patentschrift enthalten sind.

(54) **CYCLOHEXENONOXIMETHER.**

(30) Priorität: **09.08.91 DE 4126479**

(43) Veröffentlichungstag der Anmeldung:
**08.06.94 Patentblatt 94/23**

(45) Bekanntmachung des Hinweises auf die Patenterteilung:
**22.11.95 Patentblatt 95/47**

(84) Benannte Vertragsstaaten:
**AT BE CH DE ES FR GB IT LI NL**

(56) Entgegenhaltungen:
**EP-A- 0 177 913**
**EP-A- 0 243 313**
**EP-A- 0 368 227**
**GB-A- 2 125 042**

(73) Patentinhaber: **BASF Aktiengesellschaft**
**Carl-Bosch-Strasse 38**
**D-67063 Ludwigshafen (DE)**

(72) Erfinder: **MISSLITZ, Ulf**
**Am Herzel 40**
**D-6730 Neustadt (DE)**
Erfinder: **MEYER, Norbert**
**Dossenheimer Weg 22**
**D-6802 Ladenburg (DE)**
Erfinder: **KAST, Juergen**
**Kastanienstrasse24**
**D-6737 Boehl-Iggelheim (DE)**
Erfinder: **BRATZ, Matthias**
**Schwabsgasse 2**
**D-6720 Speyer (DE)**
Erfinder: **HARREUS, Albrecht**
**Teichgasse 13**
**D-6700 Ludwigshafen (DE)**
Erfinder: **WALTER, Helmut**
**Gruenstadter Strasse 82**
**D-6719 Obrigheim (DE)**
Erfinder: **WESTPHALEN, Karl-Otto**
**Mausbergweg 58**
**D-6720 Speyer (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

Erfinder: **GERBER, Matthias**
**Ritterstrasse 3**
**D-6704 Mutterstadt (DE)**

## Beschreibung

Die vorliegende Erfindung betrifft neue Cyclohexenonoximether der allgemeinen Formel I

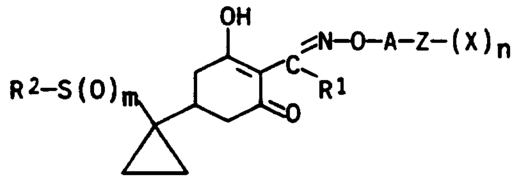

I

in der die Variablen folgende Bedeutung haben:

n 0 bis 5;

m 0 bis 2;

$R^1$ eine $C_1$-$C_6$-Alkylgruppe;

$R^2$ eine $C_1$-$C_4$-Alkylgruppe oder die Benzylgruppe;

A eine $C_1$-$C_6$-Alkylen-, eine $C_3$-$C_6$-Alkenylen- oder eine $C_3$-$6_6$-Alkinylengruppe, wobei diese Gruppen ein bis drei $C_1$-$C_3$-Alkylgruppen und/oder Halogenatome tragen können, eine gewünschtenfalls mit ein bis drei $C_1$-$C_3$-Alkylgruppen substituierte $C_3$-$C_6$-Alkylen- oder $C_4$-$C_6$-Alkenylengruppe, in der eine Methylengruppe durch ein Sauerstoff-, ein Schwefelatom, eine Sulfoxid-, eine Sulfongruppe oder eine Gruppe -N($R^a$)-, worin

$R^a$ Wasserstoff, eine $C_1$-$C_4$-Alkylgruppe, eine $C_3$-$C_6$-Alkenylgruppe oder eine $C_3$-$C_6$-Alkinylgruppe bedeutet,

ersetzt ist;

Z Phenyl, ein 5-gliedriger Heteroaromat mit ein bis drei Stickstoffatomen und/oder einem Sauerstoff- oder Schwefelatom, ein 6-gliedriger Heteroaromat mit ein bis vier Stickstoffatomen;

X Nitro, Cyano, Halogen, $C_1$-$C_4$-Alkyl, partiell oder vollständig halogeniertes $C_1$-$C_4$-Alkyl, $C_1$-$C_4$-Alkoxy, $C_1$-$C_4$-Alkylthio, partiell oder vollständig halogeniertes $C_1$-$C_4$-Alkoxy, Carboxyl, $C_1$-$C_4$-Alkoxycarbonyl, Benzyloxycarbonyl, Phenyl, Pyridyl, wobei die aromatischen Reste unsubstituiert sind oder ein bis drei der folgenden Substituenten tragen:
Nitro, Cyano, Halogen, $C_1$-$C_4$-Alkyl, partiell oder vollständig halogeniertes $C_1$-$C_4$-Alkyl, $C_1$-$C_4$-Alkoxy, $C_1$-$C_4$-Alkylthio, partiell oder vollständig halogeniertes $C_1$-$C_4$-Alkoxy, Carboxyl, $C_1$-$C_4$-Alkoxycarbonyl, Benzyloxycarbonyl, oder eine Aminogruppe -$NR^bR^c$, worin

$R^b$ Wasserstoff, eine $C_1$-$C_4$-Alkylgruppe, eine $C_3$-$C_6$-Alkenylgruppe oder eine $C_3$-$C_6$-Alkinylgruppe und

$R^c$ Wasserstoff, eine $C_1$-$C_4$-Alkylgruppe, eine $C_3$-$C_6$-Alkenylgruppe, eine $C_3$-$C_6$-Alkinylgruppe, eine $C_1$-$C_6$-Acylgruppe oder eine Benzoylgruppe, die unsubstituiert sind oder ihrerseits ein bis drei der folgenden Reste tragen: Nitro, Cyano, Halogen, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy und/oder $C_1$-$C_4$-Alkoxycarbonyl bedeuten,

sowie ihre landwirtschaftlich nutzbaren Salze und Ester mit $C_1$-$C_{10}$-Carbonsäuren oder anorganischen Säuren.

Außerdem betrifft die Erfindung herbizide Mittel, welche diese Verbindungen als wirksame Substanzen enthalten sowie ein Verfahren zur Bekämpfung von unerwünschtem Pflanzenwuchs.

Aus der Literaur sind bereits herbizid wirksame Cyclohexandione der allgemeinen Formel I'

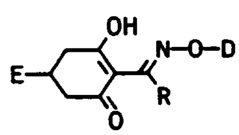

I'

bekannt, wobei E und D u.a. für die folgenden Bedeutungen stehen:

- US 4,440,566 (D = Benzyl; E = 2-Ethylthiopropyl);
- EP-A 238 021 und EP-A 125 094 (D = Benzyl, But-2-enyl; E = substituierter 5-gliedriger Heteroarylrest);
- EP-A 80 301 (D = Benzyl, But-2-enyl; E = substituiertes Phenyl);
- EP-A 177 913 (D = 2-Thenyl, 3-Thenyl; E = substituierter 5- bis 7-gliedriger Heterocyclus);

- US 4,432,786 (D = 5-Chlor-2-thenyl; E = substituiertes Phenyl);
- EP-A 243 313 (D = (E)-3-Chlorprop-2-enyl; E = 1-Methylthiocycloprop-1-yl).

Des weiteren werden in der DE-A 38 38 309 allgemein herbizid wirksame Cyclohexenonoximether vom Typ der Verbindungen I beschrieben, in der u.a. E einen substituierten 5- bis 7-gliedrigen Heterocyclus und D einen substituierten 4-Phenylbutylen- oder 4-Phenylbutenylenrest bedeuten.

Der Erfindung lag die Aufgabe zugrunde, Cyclohexenonoximether zu synthetisieren, die gegenüber den bekannten Vertretern dieser Stoffklasse eine größere Selektivität bei der Bekämpfung von Ungräsern in grasartigen Kulturen wie Reis und Mais aufweisen.

Demgemäß wurden die eingangs definierten Cyclohexenonoximether I gefunden, die eine gute herbizide Wirkung vorzugsweise gegen Arten aus der Familie der Gräser (Gramineen) besitzen. Sie sind verträglich und somit selektiv in breitblättrigen Kulturen sowie in monokotylen Gewächsen, welche nicht zu den Gramineen zählen. Ferner sind Verbindungen darunter, welche sich auch in Gramineenkulturen selektiv verhalten und gleichzeitig unerwünschte Gräser bekämpfen.

Die Cyclohexenonoximether der Formel I sind auf verschiedene Weise erhältlich, und zwar bevorzugt in an sich bekannter Weise aus schon bekannten Derivaten der Formel II (EP-A-243 313) und den entsprechenden Hydroxylaminen der Formel III (EP-A 169 521).

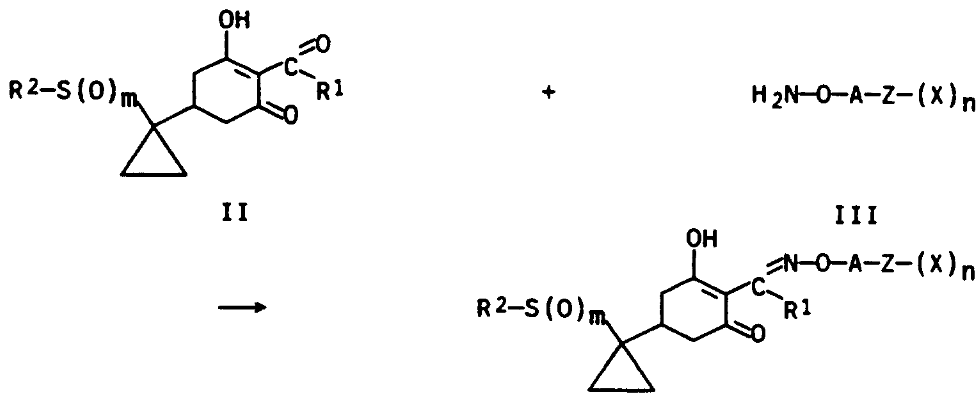

II III

I

Zweckmäßig führt man die Umsetzung in heterogener Phase in einem Lösungsmittel, bei einer ausreichenden Temperatur unterhalb von etwa 80 °C, in Gegenwart einer Base durch, wobei das Hydroxylamin III vorzugsweise als Ammoniumsalz eingesetzt wird.

Geeignete Basen sind z.B. Carbonate, Hydrogencarbonate, Acetate, Alkoholate oder Oxide von Alkali- oder Erdalkalimetallen, insbesondere Natriumhydroxid, Kaliumhydroxid, Magnesiumoxid, Calciumoxid, des weiteren organische Basen wie Pyridin und tertiäre Amine wie Triethylamin. Die Base wird beispielsweise in einer Menge von 0,5 bis 2 Mol-Äquivalent, bezogen auf die Ammoniumverbindung, zugesetzt.

Als Lösungsmittel eignen sich beispielsweise Dimethylsulfoxid; Alkohole wie Methanol, Ethanol und Isopropanol; aromatische Kohlenwasserstoffe wie Benzol und Toluol; chlorierte Kohlenwasserstoffe wie Chloroform und 1,2-Dichlorethan; aliphatische Kohlenwasserstoffe wie Hexan und Cyclohexan; Ester wie Essigsäureethylester und Ether wie Diethylether, Dioxan und Tetrahydrofuran. Vorzugsweise führt man die Umsetzung in Methanol mit Natriumhydrogencarbonat als Base durch.

Die Reaktion ist nach wenigen Stunden beendet. Das Produkt kann z.B. durch Einengen der Mischung, Verteilung des Rückstandes in Methylenchlorid/ Wasser und Abdestillieren des Lösungsmittels unter vermindertem Druck isoliert werden.

Man kann für diese Umsetzung aber auch unmittelbar die freie Hydroxylaminbase, z.B. in Form einer wäßrigen Lösung, verwenden; je nach verwendetem Lösungsmittel für die Verbindung II erhält man ein ein- oder zweiphasiges Reaktionsgemisch.

Geeignete Lösungsmittel für diese Variante sind beispielsweise Alkohole wie Methanol, Ethanol, Isopropanol und Cyclohexanol; aliphatische und aromatische, gegebenenfalls chlorierte Kohlenwasserstoffe wie Hexan, Cyclohexan, Methylenchlorid, Toluol und Dichlorethan; Ester wie Essigsäureethylester; Nitrile wie Acetonitril und cyclische Ether wie Dioxan und Tetrahydrofuran.

Besondere Bedingungen bezüglich des Drucks sind nicht nötig; im allgemeinen arbeitet man daher bei Normaldruck.

Die erfindungsgemäßen Cyclohexenonoximether I haben offensichtlich sauren Charakter, d.h. sie können Salze von Alkali- oder Erdalkalimetallverbindungen sowie Enolester bilden.

Alkalimetallsalze der Verbindungen I können durch Behandeln der 3-Hydroxyverbindungen mit Natrium- oder Kaliumhydroxid bzw. -alkoholat in wäßriger Lösung oder in einem organischen Lösungsmittel wie Methanol, Ethanol, Aceton und Toluol erhalten werden.

Andere Metallsalze wie Mangan-, Kupfer-, Zink-, Eisen-, Calcium-, Magnesium- und Bariumsalze können aus den Natriumsalzen in üblicher Weise hergestellt werden, ebenso Ammonium- und Phosphoniumsalze mittels Ammoniak, Phosphonium-, Sulfonium- oder Sulfoxoniumhydroxiden.

Zu den Hydroxylaminen III gelangt man in der Regel über eine Reihe bekannter Verfahrensschritte ausgehend von bekannten Vorprodukten:

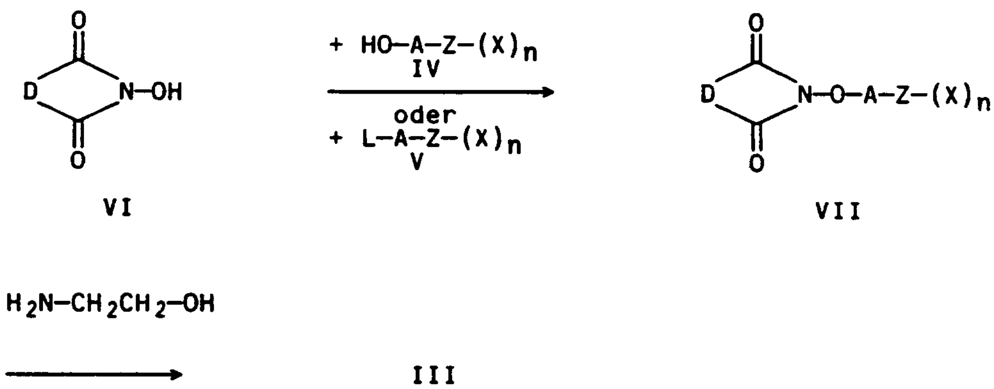


VI VII III

L = eine Abgangsgruppe, z.B. Halogen wie Chlor, Brom und Jod oder $CH_3SO_2$-O-.

Die zur Synthese der neuen Hydroxylamine der Formel III benötigten Alkylierungsmittel sind nach an sich bekannten Methoden herstellbar [vgl. "Umlagerung von Cyclopropylhetarylcarbinolen": J. Heterocycl. Chem. 14, 525 (1976), JP 55 051 004, JP 55 047 601; Houben-Weyl: Methoden der Organischen Chemie, Band 4/3, S. 424ff.; "Kupplung metallierter Heterocyclen mit 1, ω-Dibromalkanen": DE-A 28 21 409 und Chem. Ber. 114, 3667 und 3674 (1981); "Heteroatom-unterbrochene Alkylierungsmittel": DE-A 34 37 919; Tetrahedron Lett. 28, 2639 (1979), Org. Synth. Coll. Vol. 1, 436 (1944); DE-A 26 54 646; DE-A 27 14 561; J. Org. Chem. 52, 3587 (1987); DE-A 94 88 71; DE-A 94 88 72; J. Med. Chem. 26, 1570 (1983); Synthesis, 675 (1983) und J. Org. Chem. 48, 4970 (1983)].

Gewünschtenfalls können die Alkylierungsmittel V auf an sich bekannte Weise aus den Carbinolen IV ["Kupplung von Aryl-, Hetaryliodiden, -bromiden mit 1,ω-Alkenolen oder 1,ω-Alkinolen in Gegenwart von Palladiumkatalysatoren": Tetrahedron Lett. 24, 4467 (1975); Tetrahedron 35, 329 (1979); Chem. Lett. 423 (1977); Houben-Weyl: Methoden der Organischen Chemie, Band 13/9B, S. 964ff] erhalten werden [vgl. Houben-Weyl: Methoden der Organischen Chemie, Band 5/3, S. 862ff und S. 899ff und dto., Band 5/4, S. 361ff.].

Vorzugsweise koppelt man das Alkylierungsmittel V, gewünschtenfalls aber auch das Carbinol IV nach der Mitsunobu-Variante [Synthesis 1, 1981; J. Med. Chem. 33, 187 (1990)], mit einem cyclischen Hydroxyimid VI und spaltet das hierbei erhaltene geschützte Hydroxylaminderivat VII zum freien Hydroxylamin III, z.B. mit 2-Aminoethanol.

In den cyclischen Hydroximiden VI steht D z.B. für $C_2$-$C_3$-Alkylen, $C_2$-Alkenylen oder einen mit bis zu 3 Doppelbindungen und gegebenenfalls 1 Stickstoffatom enthaltenden 5- oder 6-Ring, z.B. für Phenylen, Pyridinylen, Cyclopentylen, Cyclohexylen oder Cyclohexenylen. Beispielsweise kommen folgende Substanzen in Betracht:

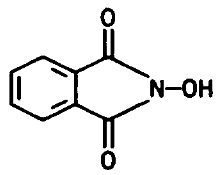
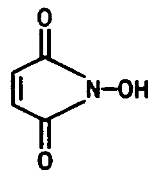
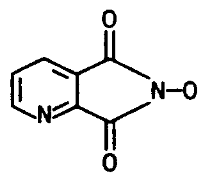

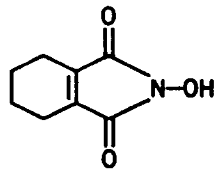
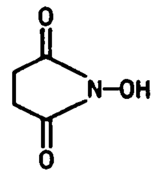
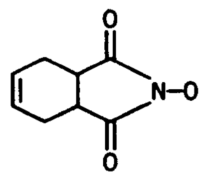

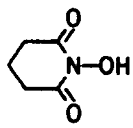

Die Umsetzung der Verbindungen V mit den Hydroxyimiden VI wird zweckmäßigerweise in Gegenwart einer Base ausgeführt. Geeignet sind prinzipiell alle Basen, die in der Lage sind, die Hydroxyimide VI zu deprotonieren ohne das Imidsystem anzugreifen. Dies sind insbesondere die sogenannten nicht-nucleophilen Basen.

Beispielsweise genannt seien Mineralbasen wie Alkalimetall- und Erdalkalimetallcarbonate, Alkalimetall- und Erdalkalimetallhydrogencarbonate, organische Basen wie aliphatische, cycloaliphatische und aromatische tertiäre Amine. Es können auch Gemische dieser Basen verwendet werden.

Als Einzelverbindungen seien folgende Basen beispielhaft aufgeführt: Natriumcarbonat, Kaliumcarbonat, Magnesiumcarbonat, Calciumcarbonat, Bariumcarbonat, die Hydrogencarbonate dieser Metalle, Trimethylamin, Triethylamin, Tributylamin, Ethyldiisopropylamin, N,N-Dimethylanilin, 4-N,N-Dimethylaminopyridin, Diazabicyclooctan, Diazabicycloundecan, N-Methylpiperidin, 1,4-Dimethylpiperazin, Pyridin, Chinolin, Bipyridin, Phenanthrolin. Bevorzugt sind die preiswerten Basen Natrium- und Kaliumcarbonat.

Die Base wird im allgemeinen in äquivalenten Mengen bis zu einem Überschuß von 5 Äquivalenten, bezogen auf das Hydroxyimid, zugegeben. Ein höherer Überschuß ist möglich, entbehrt aber zusätzliche Vorteile. Die Verwendung einer geringen Basenmenge ist ebenfalls möglich. Bevorzugt wird jedoch eine Basenmenge von 1 bis 3, insbesondere von 1 bis 2 Äquivalenten, bezogen auf das Hydroxyimid VI eingesetzt.

Die Verwendung von nucleophilen Basen, z. B. Alkalimetall- und Erdalkalimetallhydroxiden, insbesondere Natrium- und Kaliumhydroxid, ist ebenfalls möglich. In diesem Falle ist es vorteilhaft, die Base in äquivalenten Mengen bezüglich des Hydroxyimids VI einzusetzen, um einem nucleophilen Angriff der Hydroxylionen auf die Carbonylfunktion der Imidgruppierung vorzubeugen.

Zweckmäßigerweise setzt man die Ausgangsverbindungen V mit den Hydroxyimiden VI in einem Lösungsmittel um, das sich unter den Reaktionsbedingungen inert verhält. Vorteilhafte Lösungsmittel sind z.B. polare-aprotische Lösungmittel wie Dimethylformamid, N-Methylpyrrolidon, Dimethylsulfoxid, Sulfolan und cyclische Harnstoffe. Die Lösungsmittelmenge ist im allgemeinen nicht kritisch.

Die Umsetzung der Ausgangsverbindungen V mit den Hydroxyimiden VI kann auch unter Anwendung der Phasentransfer-Katalyse ausgeführt werden. In diesem Falle werden mit Wasser zwei Phasen bildende Lösungsmittel, bevorzugt Chlorkohlenwasserstoffe, eingesetzt. Als Phasentransferkatalysatoren eignen sich die üblicherweise zu solchen Zwecken verwendeten quartären Ammonium- und Phosphoniumsalze, Polyethylenglykole, Polyethylenglykolether und Kronenether, wie sie z.B. in Dehmlow et al., Phase Transfer Catalysis, S. 37-45 und S. 86-93, Verlag Chemie, Weinheim 1980, beschrieben sind.

Die Phasentransferkatalysatoren werden zweckmäßigerweise in Mengen von 1 bis 10 Vol%, bevorzugt in Mengen von 3 bis 5 Vol%, bezogen auf das Volumen der Reaktionsmischung, eingesetzt.

Die Umsetzung der Ausgangsverbindungen V mit den Hydroxyimiden VI wird im allgemeinen im Temperaturbereich zwischen 0 und 140 °C, bevorzugt zwischen 2 und 100 °C, insbesondere zwischen 40 und 80 °C, durchgeführt. Zweckmäßigerweise wird dabei so vorgegangen, daß man das Hydroxyimid VI zusammen mit der Base im Lösungsmittel vorlegt und das Ausgangsmaterial V zu dieser Lösung dosiert. Dabei kann es sich als günstig erweisen, wenn das Hydroxyimid bei einer tieferen Temperatur, beispielsweise bei 0 bis 50 °C, zugegeben und die Reaktionsmischung erst nach dieser Zugabe auf die eigentliche

Reaktionstemperatur erhitzt wird.

Nach beendeter Reaktion wird die abgekühlte Reaktionsmischung zweckmäßigerweise mit Wasser versetzt, wobei sich die gebildeten Hydroxylaminderivate VII als kristalline Festkörper oder als Öle abscheiden. Die auf diese Weise erhaltenen Hydroxylaminderivate können, falls gewünscht, durch Umkristallisation oder durch Extraktion weiter gereinigt werden.

Die Hydroxylaminderivate VII können zwischengelagert werden oder sogleich in die Hydroxylamine III (mit freier Aminogruppe) umgewandelt werden.

Diese Umwandlung kann nach an sich bekannten Verfahren durchgeführt werden, wie sie beispielsweise in DE-A 36 15 973 und den darin zitierten Schriften beschrieben sind. Bevorzugt wird das Verfahren gemäß DE-A 36 15 973 angewandt, nachdem die Hydroxylamine III mittels Ethanolamin freigesetzt werden. Die Freisetzung der Hydroxylamine III mit Hilfe anderer Basen wie wäßrigen Mineralbasen, mit Aminen, Hydrazinen, Hydroxylaminen oder mittels wäßriger Säuren ist ebenfalls möglich.

Aus den nach diesen Verfahren erhaltenen Reaktionsgemischen können die Hydroxylaminderivate III mittels üblicher Aufarbeitungsmethoden isoliert weren, beispielsweise durch Extraktion oder durch Kristallisation. Zur Erhöhung der Kristallisationstendenz dieser Hydroxylamine kann es oftmals förderlich sein, diese in ihre Salze mit Mineralsäuren oder organischen Säuren überzuführen. Dazu werden im allgemeinen verdünnte Lösungen dieser Säuren mit den Hydroxylaminen umgesetzt, und zwar zweckmäßigerweise in etwa äquivalenten Mengen. Die erhaltenen Hydroxylammoniumsalze können wie die Hydroxylamine III mit freier Aminogruppe direkt zu den Herbiziden der Formel I weiterverarbeitet werden oder auch, falls gewünscht, gelagert werden.

Die Cyclohexenonoximether I können bei der Herstellung als Isomerengemische anfallen, wobei sowohl E-/Z-Isomerengemische als auch Enantiomeren- oder Diastereoisomerengemische möglich sind. Die Isomerengemische können gewünschtenfalls nach den hierfür üblichen Methoden, z.B. durch Chromatographie oder durch Kristallisation, getrennt werden.

Die Cyclohexenonoximether I können in mehreren tautomeren Formen geschrieben werden, die alle von der Erfindung umfaßt werden.

Im Hinblick auf die biologische Wirksamkeit werden Cyclohexenonoximether der Formel I bevorzugt, in denen die Substituenten folgende Bedeutung haben:

n 0 bis 5, insbesondere 0, 1 oder 2 oder 0 bis 5 für den Fall, daß Z Phenyl und alle X Halogen bedeuten. Bedeutet Z einen Heterocyclus, so entspricht der maximal mögliche Wert von n der Anzahl substituierbarer Ringglieder. Bei mehreren Resten X können die Substituenten gleich oder verschieden sein;

m 0 bis 2, insbesondere 0;

$R^1$ $C_1$-$C_6$-Alkyl wie Methyl, Ethyl, Propyl, n-Butyl, insbesondere Ethyl und Propyl;

$R^2$ $C_1$-$C_4$-Alkyl wie Methyl, Ethyl, Propyl, n-Butyl, insbesondere Methyl;

A $C_2$-$C_6$-Alkylen, $C_3$-$C_6$-Alkenylen oder $C_3$-$C_6$-Alkinylen wie Methylen, Ethylen, Propylen, Butylen, Pentylen, Hexylen; Propenylen, Prop-2-enylen, Butenylen, But-2-enylen, But-3-enylen, Pentenylen, Pent-2-enylen, Pent-3-enylen, Pent-4-enylen, Hexenylen, Hex-2-enylen, Hex-3-enylen, Hex-4-enylen, Hex-5-enylen; Prop-2-inylen, But-2-inylen, But-3-inylen, Pent-2-inylen, Pent-3-inylen, Pent-4-inylen, Hex-2-inylen, Hex-3-inylen, Hex-4-inylen, Hex-5-inylen bedeuten und durch ein bis drei Methyl- bzw. Ethylreste und/oder Fluor- oder Chloratome substituiert sein können; bei den ungesättigten Ketten können sowohl die cis- als auch die trans-Form auftreten. Insbesondere bevorzugt sind Propylen, Butylen, Prop-2-enylen, But-2-enylen, But-3-enylen und But-3-inylen;

$C_3$-$C_6$-Alkylen oder $C_4$-$C_6$-Alkenylen, in dem eine Methylengruppe durch ein Sauerstoff-, ein Schwefelatom, eine Sulfoxid-, eine Sulfongruppe oder -N($R^a$)- ersetzt ist, wie 3-Oxapropylen, 3-Azapropylen, 3-Thiapropylen, 3-Thiapropylen-3-oxid, 3-Thiapropylen-3,3-dioxid, 3-Oxabutylen, 3-Azabutylen, 3-Thiabutylen, 3-Thiabutylen-3-oxid, 3-Thiabutylen-3,3-dioxid, 4-Oxabutylen, 4-Azabutylen, 4-Thiabutylen, 4-Thiabutylen-4-oxid, 4-Thiabutylen-4,4-dioxid, 4-Oxabut-2-enylen, 4-Azabut-2-enylen, 4-Thiabut-2-enylen, 3-Oxapentylen, 3-Azapentylen, 3-Thiapentylen, 3-Thiapentylen-3-oxid, 3-Thiapentylen-3,3-dioxid, 4-Oxapentylen, 4-Azapentylen, 4-Thiapentylen, 4-Thiapentylen-4-oxid, 4-Thiapentylen-4,4-dioxid, 5-Oxapentylen, 5-Azapentylen, 5-Thiapentylen, 5-Thiapentylen-5-oxid, 5-Thiapentylen-5,5-dioxid, 5-Oxapent-3-enylen, 5-Azapent-3-enylen, 5-Thiapent-3-enylen, 3-Oxahexylen, 3-Azahexylen, 3-Thiahexylen, 3-Thiahexylen-3-oxid, 3-Thiahexylen-3,3-dioxid, 4-Oxahexylen, 4-Azahexylen, 4-Thiahexylen, 4-Thiahexylen-4-oxid, 4-Thiahexylen-4,4-dioxid, 5-Oxahexylen, 5-Azahexylen, 5-Thiahexylen, 5-Thiahexylen-5-oxid, 5-Thiahexylen-5,5-dioxid, 6-Oxahexylen, 6-Azahexylen, 6-Thiahexylen, 6-Thiahexylen-6-oxid, 6-Thiahexylen-6,6-dioxid, 6-Oxahex-4-enylen, 6-Azahex-4-enylen, 6-Thiahex-4-enylen bedeuten und durch ein bis drei Methyl- bzw. Ethylreste substituiert sein können; bei den ungesättigten Ketten können sowohl die cis- als auch die trans-Form auftreten. Insbesondere bevorzugt sind 3-Oxapropylen, 2-Methyl-3-oxapropylen, 3-Oxabutylen und 4-Oxabutylen;

$R^a$ Wasserstoff;

$C_1$-$C_4$-Alkyl wie Methyl, Ethyl, n-Propyl, 1-Methylethyl, n-Butyl, 1-Methylpropyl, 2-Methylpropyl und 1,1-Dimethylethyl, insbesondere Methyl und Ethyl;
$C_3$-$C_6$-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,2-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl, insbesondere 2-Propenyl und 2-Butenyl;
$C_3$-$C_6$-Alkinyl wie 2-Propinyl, 2-Butinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl, insbesondere 2-Propinyl und 2-Butinyl;
Z Phenyl
5-gliedriges Heteroaryl wie Furanyl, Pyrrolyl, Thienyl, Imidazolyl, Pyrazolyl, Isoxazolyl, Oxazolyl, Isothiazolyl, Thiazolyl, Oxadiazolyl, Thiadiazolyl, Triazolyl, insbesondere Furanyl und Thienyl,
6-gliedriges Heteroaryl wie Pyridyl, Pyridazyl, Pyrimidyl, Pyrazyl, Triazyl, Tetrazyl, insbesondere Pyridyl und Pyrimidyl;
X Nitro, Cyano,
Halogen wie Fluor, Chlor, Brom und Iod, insbesondere Fluor und Chlor;
$C_1$-$C_4$-Alkyl wie Methyl, Ethyl, n-Propyl, 1-Methylethyl, n-Butyl, 1-Methyl-propyl, 2-Methylpropyl und 1,1-Dimethylethyl, insbesondere Methyl und 1,1-Dimethylethyl,
$C_1$-$C_4$-Alkoxy wie Methoxy, Ethoxy, n-Propoxy, 1-Methylethoxy, n-Butoxy, 1-Methylpropoxy, 2-Methylpropoxy und 1,1-Dimethylethoxy, insbesondere Methoxy, Ethoxy, 1-Methylethoxy und 1,1-Dimethylethoxy,
$C_1$-$C_4$-Alkylthio wie Methylthio, Ethylthio, Propylthio, 1-Methylethylthio, Butylthio, 1-Methylpropylthio, 2-Methylpropylthio und 1,1-Dimethylethylthio, insbesondere Methylthio und Ethylthio,
$C_1$-$C_4$-Halogenalkyl wie Fluormethyl, Difluormethyl, Trifluormethyl, Chlordifluormethyl, Dichlorfluormethyl, Trichlormethyl, 1-Fluor-ethyl, 2-Fluorethyl, 2,2-Difluorethyl, 2,2,2-Trifluorethyl, 2-Chlor-2,2-difluorethyl, 2,2-Dichlor-2-fluorethyl, 2,2,2-Trichlorethyl, Pentafluorethyl und 3-Chlorpropyl, insbesondere Difluormethyl, Trifluormethyl, 2,2,2-Trifluorethyl und Pentafluorethyl,
$C_1$-$C_4$-Halogenalkoxy wie Difluormethoxy, Trifluormethoxy, Chlordifluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere Trifluormethoxy,
Carboxyl,
$C_1$-$C_4$-Alkoxycarbonyl wie Methoxycarbonyl, Ethoxycarbonyl, n-Propoxycarbonyl, 1-Methylethoxycarbonyl, n-Butoxycarbonyl und 1,1-Dimethylethoxycarbonvl, insbesondere Methoxycarbonyl, Ethoxycarbonyl und 1,1-Dimethylethoxycarbonyl, insbesondere Methoxycarbonyl sowie
Benzyloxycarbonyl, Phenyl und Pyridyl, wobei die aromatischen Reste jeweils unsubstituiert sein oder ihrerseits ein bis drei der folgenden Reste tragen können: Nitro, Cyano, Carboxyl, Benzyloxycarbonyl, Halogen wie im allgemeinen und im besonderen vorstehend genannt; $C_1$-$C_4$-Alkyl wie vorstehend genannt, insbesondere Methyl, Ethyl und 1-Methylethyl; $C_1$-$C_4$-Alkoxy wie vorstehend genannt, insbesondere Methoxy und Ethoxy; $C_1$-$C_4$-Alkylthio wie vorstehend genannt, insbesondere Methylthio; $C_1$-$C_4$-Halogenalkyl wie vorstehend genannt, insbesondere Trifluormethyl; $C_1$-$C_4$-Halogenalkoxy wie vorstehend genannt, insbesondere Difluormethoxy und Trifluormethoxy und/oder $C_1$-$C_4$-Alkoxycarbonyl wie vorstehend genannt, insbesondere Methoxycarbonyl und Ethoxycarbonyl,
eine Aminogruppe $-NR^bR^c$:

$R^b$ Wasserstoff, $C_1$-$C_4$-Alkyl, $C_3$-$C_6$-Alkenyl und $C_3$-$C_6$-Alkinyl wie für $R^a$ im allgemeinen und im besonderen genannt;

$R^c$ Wasserstoff, $C_1$-$C_4$-Alkyl, $C_3$-$C_6$-Alkenyl und $C_3$-$C_6$-Alkinyl wie für $R^a$ im allgemeinen und im besonderen genannt;
$C_1$-$C_6$-Acylgruppen wie Acetyl, Propionyl, Butyryl, 2-Methylpropionyl, Pentanoyl, 2-Methylbutyryl, 3-Methylbutyryl, 2,2-Dimethylpropionyl₁ Hexanoyl, 2-Methylpentanoyl, 3-Methylpentanoyl, 4-Methylpentanoyl, 2,2-Dimethylbutyryl, 2,3-Dimethylbutyryl, 3,3-Dimethylbutyryl und 2-Ethylbutytryl,

insbesondere Acetyl und Propionyl; oder Benzoyl, wobei der Aromat unsubstituiert ist oder noch ein bis drei Reste trägt, ausgewählt aus einer Gruppe bestehend aus: Nitro, Cyano, Carboxyl, Benzyloxycarbonyl, Halogen wie im allgemeinen und im besonderen vorstehend genannt; $C_1$-$C_4$-Alkyl wie vorstehend genannt, insbesondere Methyl; $C_1$-$C_4$-Alkoxy wie vorstehend genannt, insbesondere Methoxy und Ethoxy; $C_1$-$C_4$-Alkylthio wie vorstehend genannt, insbesondere Methylthio; $C_1$-$C_4$-Halogenalkyl wie vorstehend genannt, insbesondere Trifluormethyl; $C_1$-$C_4$-Halogenalkoxy wie vorstehend genannt, insbesondere Difluormethoxy und Trifluormethoxy und $C_1$-$C_4$-Alkoxycarbonyl wie vorstehend genannt insbesondere Methoxycarbonyl und Ethoxycarbonyl.

Besonders bevorzugte Cyclohexenonoximether der Formel I sind der folgenden Tabelle 1 zu entnehmen.

Tabelle 1

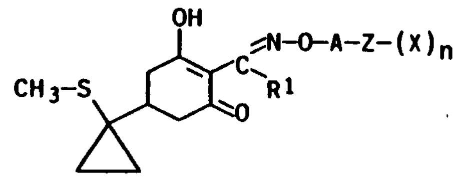

I

$(R^2-S(O)_m- = CH_3-S-)$

| | $R^1$ | A | Z $(X)_n$ | physikalische Daten ($^1$H-NMR [ppm]; Fp. [°C]) |
|---|---|---|---|---|
| 1.01 | Ethyl | trans-$CH_2CH_2CH{=}CH$- | 4-Fluorphenyl | 2,13 (s,3H); 6,10 (dt,1H); 6,43 (d,1H); 7,00 (m,2H); 7,30 (m,2H) |
| 1.02 | n-Propyl | trans-$CH_2CH_2CH{=}CH$- | 4-Fluorphenyl | 2,15 (s,3H); 6,10 (dt,1H); 6,45 (d,1H); 7,00 (m,2H); 7,30 (m,2H); |
| 1.03 | Ethyl | trans-$CH_2CH_2CH{=}CH$- | 4-Chlorphenyl | 2,13 (s,3H); 6,20 (dt,1H); 6,43 (d,1H); 7,27 (s,4H) |
| 1.04 | n-Propyl | trans-$CH_2CH_2CH{=}CH$- | 4-Chlorphenyl | 2,15 (s,3H); 6,20 (dt,1H); 6,45 (d,1H); 7,30 (s,4H) |
| 1.05 | Ethyl | -$CH_2CH_2CH_2CH_2$- | 4-Fluorphenyl | 2,13 (s,3H); 6,95 (m,2H); 7,10 (m,2H) |
| 1.06 | n-Propyl | -$CH_2CH_2CH_2CH_2$- | 4-Fluorphenyl | 2,15 (s,3H); 4,10 (m,2H); 6,95 (m,2H); 7,10 (m,2H) |
| 1.07 | Ethyl | -$CH_2CH_2$-O- | 4-Fluorphenyl | 2,13 (s,3H), 6,80-7,10 (m,2H) |
| 1.08 | n-Propyl | -$CH_2CH_2$-O- | 4-Fluorphenyl | 2,15 (s,3H); 4,20 (m,2H); 4,43 (m,2H); 6,80-7,10 (m,4H) |
| 1.09 | Ethyl | -$CH_2CH_2CH_2$-O- | 4-Fluorphenyl | 2,13 (s,3H), 6,90 (m,2H); 7,00 (m,2H) |

Tabelle 1 (Fortsetzung)

| | $R^1$ | A | Z (X)$_n$ | physikalische Daten ($^1$H-NMR [ppm]; Fp. [°C]) |
|---|---|---|---|---|
| 1.10 | n-Propyl | $-CH_2CH_2CH_2-O-$ | 4-Fluorphenyl | 2,15 (s,3H); 4,05 (m,2H); 4,25 (m,2H); 6,85 (m,2H); 6,95 (m,2H) |
| 1.11 | Ethyl | $-CH_2CH(CH_3)-O-$ | 4-Chlorphenyl | 2,13 (s,3H); 4,20 (m,2H); 4,60 (m,1H); 6,90 (d,2H); 7,20 (d,2H) |
| 1.12 | n-Propyl | $-CH_2CH(CH_3)-O-$ | 4-Chlorphenyl | 2,15 (s,3H); 4,20 (m,2H); 4,65 (m,1H); 6,90 (d,2H); 7,25 (d,2H) |
| 1.13 | Ethyl | $-CH_2CH_2CH_2CH_2-$ | 2-Thienyl | 2,13 (s,3H); 4,10 (m,2H); 6,80 (m,1H); 6,95 (m,1H); 7,15 (m,1H) |
| 1.14 | n-Propyl | $-CH_2CH_2CH_2CH_2-$ | 2-Thienyl | 2,15 (s,3H); 4,10 (m,2H); 6,80 (m,1H); 6,95 (m,1H); 7,15 (m,1H) |
| 1.15 | Ethyl | $-CH_2-$ | 5-Chlor-2-thienyl | 2,13 (s,3H); 5,10 (s,2H); 6,85 (m,2H) |
| 1.16 | n-Propyl | $-CH_2-$ | 5-Chlor-2-thienyl | 2,15 (s,3H); 5,13 (s,2H); 6,83 (m,2H) |
| 1.17 | Ethyl | $-CH_2-$ | 3-Methyl-5-isoxazolyl | 2,15 (s,3H); 2,35 (s,3H); 5,10 (s,2H); 6,15 (s,1H) |
| 1.18 | n-Propyl | $-CH_2-$ | 3-Methyl-5-isoxazolyl | 2,15 (s,3H); 2,35 (s,3H); 5,10 (s,2H); 6,15 (s,1H) |
| 1.19 | Ethyl | $-CH_2CH_2OCH_2-$ | 3-Methylphenyl | 2,13 (s,3H); 2,35 (s,3H); 4,55 (s,2H); 7,05-7,30 (m,4H) |
| 1.20 | n-Propyl | $-CH_2CH_2OCH_2-$ | 3-Methylphenyl | 2,13 (s,3H); 2,35 (s,3H); 4,55 (s,2H); 7,05-7,30 (m,4H) |
| 1.21 | Ethyl | cis-$CH_2C(CH_3)=CH-$ | 4-Chlorphenyl | 1,90 (s,3H); 2,15 (s,3H); 4,60 (s,2H); 6,55 (s,1H); 7,15-7,35 (m,4H) |
| 1.22 | n-Propyl | cis-$CH_2C(CH_3)=CH-$ | 4-Chlorphenyl | 1,90 (s,3H); 2,15 (s,3H); 4,60 (s,2H); 6,55 (s,1H); 7,15-7,35 (m,4H) |

Tabelle 1 (Fortsetzung)

| | $R^1$ | A | Z $(X)_n$ | physikalische Daten ($^1$H-NMR [ppm]; Fp. [°C]) |
|---|---|---|---|---|
| 1.23 | Ethyl | $-CH_2CH_2CH_2-O-$ | 2,4-Dichlorphenyl | 2,15 (s,3H); 4,10 (m,2H); 4,30 (m,2H); 6,85 (d,1H); 7,15 (dd,1H); 7,35 (d,1H) |
| 1.24 | n-Propyl | $-CH_2CH_2CH_2-O-$ | 2,4-Dichlorphenyl | 2,15 (s,3H); 4,10 (m,2H); 4,30 (m,2H); 6,85 (d,1H); 7,15 (dd,1H); 7,35 (d,1H) |
| 1.25 | Ethyl | $-CH_2CH_2-O-$ | 4-Chlorphenyl | 2,13 (s,3H); 4,20 (m,2H); 4,40 (m,2H); 6,85 (d,2H); 7,25 (d,2H) |
| 1.26 | n-Propyl | $-CH_2CH_2-O-$ | 4-Chlorphenyl | 2,13 (s,3H); 4,20 (m,2H); 4,40 (m,2H); 6,85 (d,2H); 7,25 (d,2H) |
| 1.27 | Ethyl | $-CH_2CH_2-O-$ | 2,4-Difluorphenyl | 2,13 (s,3H); 4,25 (m,2H); 4,40 (m,2H); 6,70-7,00 (m,3H) |
| 1.28 | n-Propyl | $-CH_2CH_2-O-$ | 2,4-Difluorphenyl | 2,13 (s,3H); 4,25 (m,2H); 4,40 (m,2H); 6,70-7,00 (m,3H) |
| 1.29 | Ethyl | $-CH_2CH_2CH_2-O-$ | 2,4-Difluorphenyl | 2,13 (s,3H); 4,10 (t,2H); 4,30 (t,2H); 6,70-7,00 (m,3H) |
| 1.30 | n-Propyl | $-CH_2CH_2CH_2-O-$ | 2,4-Difluorphenyl | 2,13 (s,3H); 4,10 (t,2H); 4,30 (t,2H); 6,70-7,00 (m,3H) |
| 1.31 | Ethyl | $-CH_2CH(CH_3)-O-$ | 4-Fluorphenyl | 2,15 (s,3H); 4,15 (m,2H); 4,55 (m,1H); 6,80-7,00 (m,4H) |
| 1.32 | n-Propyl | $-CH_2CH(CH_3)-O-$ | 4-Fluorphenyl | 2,15 (s,3H); 4,15 (m,2H); 4,55 (m,1H); 6,80-7,00 (m,4H) |
| 1.33 | Ethyl | $-CH_2CH_2-O-$ | 2,4-Dichlorphenyl | 2,15 (s,3H); 4,25 (m,2H); 4,45 (m,2H); 6,85 (d,1H), 7,15 (dd,1H), 7,35 (d,1H) |
| 1.34 | n-Propyl | $-CH_2CH_2-O-$ | 2,4-Dichlorphenyl | 2,15 (s,3H); 4,25 (m,2H); 4,45 (m,2H); 6,85 (d,1H), 7,15 (dd,1H), 7,35 (d,1H) |

Tabelle 1 (Fortsetzung)

| | $R^1$ | A | Z $(X)_n$ | physikalische Daten ($^1H$-NMR [ppm]; Fp. [°C]) |
|---|---|---|---|---|
| 1.35 | n-Propyl | $-CH_2CH_2CH_2-O-$ | 4-Chlorphenyl | 2,15 (s,3H); 4,05 (t,2H); 4,25 (t,2H); 6,80 (m,2H), 7,20 (m,2H) |

Tabelle 2

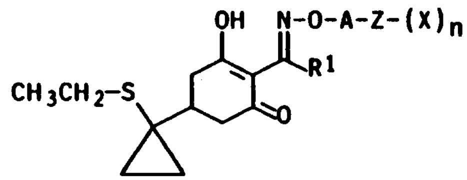

I

$(R^2\text{-}S(O)_m\text{-} = CH_3CH_2\text{-}S\text{-})$

| | $R^1$ | A | Z $(X)_n$ | physikalische Daten ($^1$H-NMR [ppm]; Fp. [°C]) |
|---|---|---|---|---|
| 2.01 | Ethyl | $-CH_2CH(CH_3)-O-$ | 4-Chlorphenyl | 1,60 (m,1H); 4,20 (m,2H); 4,65 (m,1H); 6,80 (d,2H); 7,25 (d,2H) |
| 2.02 | n-Propyl | $-CH_2CH(CH_3)-O-$ | 4-Chlorphenyl | 1,60 (m,1H); 4,20 (m,2H); 4,65 (m,1H); 6,80 (d,2H); 7,25 (d,2H) |
| 2.03 | Ethyl | $-CH_2CH_2-O-$ | 2,4-Difluorphenyl | 1,60 (m,1H); 4,25 (m,2H); 4,40 (m,2H); 6,70-7,00 (m,3H) |
| 2.04 | n-Propyl | $-CH_2CH_2-O-$ | 2,4-Difluorphenyl | 1,60 (m,1H); 4,25 (m,2H); 4,40 (m,2H); 6,70-7,00 (m,3H) |
| 2.05 | Ethyl | $-CH_2CH_2CH_2-O-$ | 2,4-Difluorphenyl | 1,60 (m,1H); 4,10 (t,2H); 4,30 (t,2H); 6,70-7,00 (m,3H) |
| 2.06 | n-Propyl | $-CH_2CH_2CH_2-O-$ | 2,4-Difluorphenyl | 1,60 (m,1H); 4,10 (t,2H); 4,30 (t,2H); 6,70-7,00 (m,3H) |
| 2.07 | Ethyl | $-CH_2CH(CH_3)-O-$ | 4-Fluorphenyl | 1,60 (m,1H); 4,20 (m,2H); 4,55 (m,1H); 6,80-7,00 (m,4H) |
| 2.08 | n-Propyl | $-CH_2CH(CH_3)-O-$ | 4-Fluorphenyl | 1,60 (m,1H); 4,20 (m,2H); 4,55 (m,1H); 6,80-7,00 (m,4H) |

Tabelle 2 (Fortsetzung)

| | $R^1$ | A | Z $(X)_n$ | physikalische Daten ($^1H$-NMR [ppm]; Fp. [°C]) |
|---|---|---|---|---|
| 2.09 | Ethyl | $-CH_2-$ | 5-Chlorthienyl | 1,60 (m,1H); 5,10 (s,2H); 6,83 (m,2H) |
| 2.10 | n-Propyl | $-CH_2-$ | 5-Chlorthienyl | 1,60 (m,1H); 5,10 (s,2H); 6,83 (m,2H) |

Tabelle 3

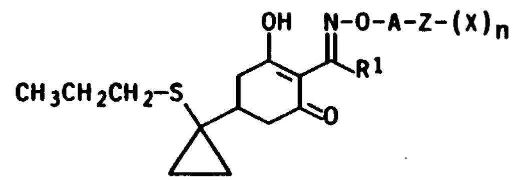

I

$(R^2-S(O)_m- = CH_3CH_2CH_2-S-)$

| | $R^1$ | A | Z $(X)_n$ | physikalische Daten ($^1$H-NMR [ppm]; Fp. [°C]) |
|---|---|---|---|---|
| 3.01 | Ethyl | $-CH_2CH(CH_3)-O-$ | 4-Chlorphenyl | 0,75 (m,2H); 4,20 (m,2H); 4,65 (m,1H); 6,80 (d,2H); 7,25 (d,2H) |
| 3.02 | n-Propyl | $-CH_2CH(CH_3)-O-$ | 4-Chlorphenyl | 0,75 (m,2H); 4,20 (m,2H); 4,65 (m,1H); 6,80 (d,2H); 7,25 (d,2H) |
| 3.03 | Ethyl | $-CH_2CH_2-O-$ | 2,4-Difluorphenyl | 0,75 (m,2H); 4,25 (m,2H); 4,40 (m,2H); 6,70-7,00 (m,3H) |
| 3.04 | n-Propyl | $-CH_2CH_2-O-$ | 2,4-Difluorphenyl | 0,75 (m,2H); 4,25 (m,2H); 4,40 (m,2H); 6,70-7,00 (m,3H) |
| 3.05 | Ethyl | $-CH_2CH_2CH_2-O-$ | 2,4-Difluorphenyl | 0,75 (m,2H); 4,10 (t,2H); 4,25 (t,2H); 6,70-7,00 (m,3H) |
| 3.06 | n-Propyl | $-CH_2CH_2CH_2-O-$ | 2,4-Difluorphenyl | 0,75 (m,2H); 4,10 (t,2H); 4,25 (t,2H); 6,70-7,00 (m,3H) |
| 3.07 | n-Propyl | $-CH_2-$ | 5-Chlorthienyl | 0,75 (m,2H); 5,10 (s,2H); 6,83 (m,2H) |

Die Cyclohexenonoximether I eignen sich als Herbizide, insbesondere zur Bekämpfung von Pflanzenarten aus der Familie der Gramineen (Gräser). Im allgemeinen sind sie verträglich und somit selektiv in breitblättrigen Kulturen sowie in monokotylen (einkeimblättrigen) Gewächsen, welche nicht zu den Gramineen zählen, insbesondere in Mais und Reis. Einigen Derivate der Verbindungen I können jedoch auch Selektivität in Gramineen aufweisen, wodurch sich unerwünschte Gräser gezielt bekämpfen lassen.

Die Cyclohexenonoximether I bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether-und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten im allgemeinen zwischen 0,01 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR-Spektrum) eingesetzt.

Beispiele für solche Zubereitungen sind:

I. eine Lösung von aus 20 Gew.-Teilen der Verbindung Nr. 1.03 in einer Mischung, bestehend aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.-Teilen des Anlagerungsproduktes von 40 Mol Ethylenoxid an 1 Mol Ricinusöl. Durch feines Verteilen des Gemisches in 100 000 Gew.-Teilen Wasser erhält man eine Dispersion, die 0,02 Gew.-% des Wirkstoffs enthält.

II. eine Dispersion von 20 Gew.-Teilen der Verbindung Nr. 1.05 in einer Mischung, bestehend aus 40 Gew.-Teilen Cyclohexanon, 30 Gew.-Teilen Isobutanol, 20 Gew.-Teilen des Anlagerungsproduktes von 70 Mol Ethylenoxid an 1 Mol Isooctylphenol und 10 Gew.-Teilen des Anlagerungsproduktes von 40 Mol Ethylenoxid an 1 Mol Ricinusöl. Die Mischung dieser Dispersion mit 100 000 Gewichtsteilen Wasser enthält 0,02 Gew.-% des Wirkstoffes.

III. eine Dispersion von 20 Gew.-Teilen der Verbindung Nr. 1.07 in einer Mischung, bestehend aus 25 Gew.-Teilen Cyclohexanon, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl. Die Mischung dieser Dispersion mit 100 000 Gew.-Teilen Wasser enthält 0,02 % des Wirkstoffes;

IV. eine in einer Hammermühle vermahlene Mischung aus 20 Gew.-Teilen der Verbindung Nr. 1.09, 3 Gew.-Teilen des Natriumsalzes der Diisobutylnaphtalin-$\alpha$-sulfonsäure, 17 Gew.-Teilen des Natriumsalzes

einer Ligninsulfonsäure aus einer Sulfit-Ablauge und 60 Gew.-Teilen pulverförmigem Kieselsäuregel. Durch feines Verteilen der Mischung in 20 000 Gew.-Teilen Wasser erhält man eine Spritzbrühe, die 0,1 Gew.-% des Wirkstoffs enthält;

V. eine innige Mischung aus 3 Gew.-Teilen der Verbindung Nr. 1.11 und 97 Gew.-Teilen feinteiligem Kaolin. Dieses Stäubemittel enthält 3 Gew.-% Wirkstoff;

VI. eine stabile ölige Dispersion aus 20 Gew.-Teilen der Verbindung Nr. 1.01, 2 Gew.-Teilen des Calciumsalzes der Dodecylbenzolsulfonsäure, 8 Gew.-Teilen Fettalkohol-polyglykolether, 2 Gew.-Teilen des Natriumsalzes eines Phenolsulfonsäure-harnstoffformaldehyd Kondensates und 68 Gew.-Teilen eines paraffinischen Mineralöls;

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 3,0, vorzugsweise 0,01 bis 1 kg/ha aktive Substanz (a.S.).

In Anbetracht der Vielseitigkeit der Applikationsmethoden können die Cyclohexenonoximether I bzw. sie enthaltende Mittel noch in einer weiteren Zahl von Kulturplfanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterrübe |
| Brassica napus var. napus | Raps |
| Brassica napus var. napobrassica | Kohlrübe |

| Botanischer Name | Deutscher Name |
|---|---|
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus sinensis | Apfelsine, Orange |
| Coffea arabica (Coffea canephora, Coffea liberica) | Kaffee |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Musa spp. | Obst- und Mehlbanane |
| Nicotiana tabacum (N. rustica) | Tabak |
| Olea europaea | Ölbaum |
| Oryza sativa | Reis |
| Phaseolus lunatus | Mondbohne |
| Phaseolus vulgaris | Buschbohnen |
| Picea abies | Rotfichte |
| Pinus spp. | Kiefer |
| Pisum sativum | Gartenerbse |
| Prunus avium | Süßkirsche |
| Prunus persica | Pfirsich |
| Pyrus communis | Birne |

| Botanischer Name | Deutscher Name |
|---|---|
| Ribes sylvestre | Rote Johannisbeere |
| Ricinus communis | Rizinus |
| Saccharum officinarum | Zuckerrohr |
| Secale cereale | Roggen |
| Solanum tuberosum | Kartoffel |
| Sorghum bicolor (s. vulgare) | Mohrenhirse |
| Theobroma cacao | Kakaobaum |
| Trifolium pratense | Rotklee |
| Triticum aestivum | Weizen |
| Triticum durum | Hartweizen |
| Vicia faba | Pferdebohnen |
| Vitis vinifera | Weinrebe |
| Zea mays | Mais |

Zur Verbreiterung des Wirkungsspektrums und zur Erzielung synergistischer Effekte können die Cyclohexenonoximether I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, die in 2-Stellung z.B. eine Carboxy- oder Carbimino-Gruppe tragen, Chinolincarbonsäurederivate, Imidazolinone, Sulfonamide, Sulfonylharnstoffe, Aryloxy-, Heteroaryloxyphenoxypropionsäuren sowie deren Salze, Ester und Amide und andere in Betracht.

Außerdem kann es von Nutzen sein, die Verbindungen I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Herstellungsbeispiele

2-[1-[4-trans-(4-Fluorphenyl)but-3-enyloxyimino]propyl]-3-hydroxy -5-(1-methylthiocyclopropyl)-2-cyclohexen-1-on (Verbindung 1.1)

Eine Mischung aus 2,0 g (7,9 mmol) 3-Hydroxy-5-(1-methylthiocyclopropyl)-2-propionyl-2-cyclohexen-1-on, 1,6 g (9,0 mmol) O-[4-trans-(4-Fluorphenyl)but-3-enyl]hydroxylamin und 100 ml Methanol wurde 24 Std. gerührt und anschließend bei reduziertem Druck eingeengt. Ausbeute: 100 %
$^1$H-NMR (200 MHz, in $CDCl_3$): $\delta$ [ppm] = 0,77 (m, 2H); 0,97 (m, 2H); 1,10 (t, 3H); 1,60 (m, 1H); 2,13 (s, 3H); 2,40-2,80 (m, 6H); 2,90 (q, 2H); 4,17 (t, 2H); 6,10 (dt, 1H); 6,43 (d, 1H); 7,00 (m, 2H); 7,30 (m, 2H); 14,80 (s, 1H).

2-[1-[4-trans-(4-Chlorphenyl)but-3-enyloxyimino]propyl]-3-hydroxy-5-(1-methylthiocyclopropyl)-2-cyclohexen-1-on (Verbindung 1.3)

Eine Mischung aus 2,0 g (7,9 mmol) 3-Hydroxy-5-(1-methylthiocyclopropyl)-2-propionyl-2-cyclohexen-1-on, 1,8 g (9,0 mmol) O-[4-trans-(4-Chlorphenyl)but-3-enyl]hydroxylamin und 100 ml Methanol wurde 24 Std. gerührt und anschließend bei reduziertem Druck eingeengt. Ausbeute: 100 %
$^1$H-NMR (200 MHz, in $CDCl_3$): $\delta$ [ppm] = 0,77 (m, 2H); 0,97 (m, 2H), 1,10 (t, 3H); 1,60 (m, 1H); 2,13 (s, 3H); 2,40-2,80 (m, 6H); 2,90 (q, 2H); 4,20 (t, 2H); 6,20 (dt, 1H); 6,43 (d, 1H), 7,27 (s, 4H); 14,70 (s, 1H).

Anwendungsbeispiele

Die herbizide Wirkung der Cyclohexenonoximether der Formel I ließ sich durch Gewächshausversuche zeigen:
Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zwecke der Nachauflaufbehandlung wurden die Testpflanzen bereits in den Versuchsgefäßen angezogen oder einige Tage vorher in die Versuchsgefäße verpflanzt. Die Applikation der in Wasser suspendierten oder emulgierten Wirkstoffe erfolgte je nach Wuchsform erst bei einer Wuchshöhe von 3 bis 15 cm. Die Aufwandmenge für die Nachauflaufbehandlung betrug 0,25 kg/ha a.S.

Die Pflanzen wurden artenspezifisch bei Temperaturen von 10-25 °C bzw. 20-35 °C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Botanischer Name | Deutscher Name | Englischer Name |
|---|---|---|
| Echinochloa crus-galli | Hühnerhirse | barnyardgrass |
| Setaria faberii | Borstenhirse | Giant foxtail |
| Setaria viridis | Grüne Borstenhirse | Green foxtail |

Das Ergebnis zeigte, daß sich mit Verbindung Nr. 1.05 grasartige Pflanzen im Nachauflaufverfahren sehr gut bekämpfen lassen.

**Patentansprüche**

**Patentansprüche für folgende Vertragsstaaten : AT, BE, CH, DE, FR, GB, IT, LI, NL**

**1.** Cyclohexenonoximether der allgemeinen Formel I

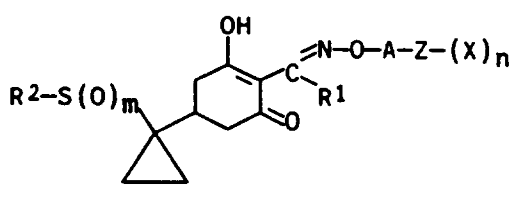

I

in der die Variablen folgende Bedeutung haben:

n 0 bis 5;

m 0 bis 2;

$R^1$ eine $C_1$-$C_6$-Alkylgruppe;

$R^2$ eine $C_1$-$C_4$-Alkylgruppe oder die Benzylgruppe;

A eine $C_1$-$C_6$-Alkylen-, eine $C_3$-$C_6$-Alkenylen- oder eine $C_3$-$C_6$-Alkinylengruppe, wobei diese Gruppen ein bis drei $C_1$-$C_3$-Alkylgruppen und/oder Halogenatome tragen können,
eine gewünschtenfalls mit ein bis drei $C_1$-$C_3$-Alkylgruppen substituierte $C_3$-$C_6$-Alkylen- oder $C_4$-$C_6$-Alkenylengruppe, in der eine Methylengruppe durch ein Sauerstoff-, ein Schwefelatom, eine Sulfoxid-, eine Sulfongruppe oder eine Gruppe -N($R^a$)-, worin

$R^a$ Wasserstoff, eine $C_1$-$C_4$-Alkylgruppe, eine $C_3$-$C_6$-Alkenylgruppe oder eine $C_3$-$C_6$-Alkinylgruppe bedeutet, ersetzt ist;

Z Phenyl, ein 5-gliedriger Heteroaromat mit ein bis drei Stickstoffatomen und/oder einem Sauerstoff-

oder Schwefelatom, ein 6-gliedriger Heteroaromat mit ein bis vier Stickstoffatomen;
X Nitro, Cyano, Halogen, $C_1$-$C_4$-Alkyl, partiell oder vollständig halogeniertes $C_1$-$C_4$-Alkyl, $C_1$-$C_4$-Alkoxy, $C_1$-$C_4$-Alkylthio, partiell oder vollständig halogeniertes $C_1$-$C_4$-Alkoxy, Carboxyl, $C_1$-$C_4$-Alkoxycarbonyl, Benzyloxycarbonyl, Phenyl, Pyridyl, wobei die aromatischen Reste unsubstituiert sind oder ein bis drei der folgenden Substituenten tragen: Nitro, Cyano, Halogen, $C_1$-$C_4$-Alkyl, partiell oder vollständig halogeniertes $C_1$-$C_4$-Alkyl, $C_1$-$C_4$-Alkoxy, $C_1$-$C_4$-Alkylthio, partiell oder vollständig halogeniertes $C_1$-$C_4$-Alkoxy, Carboxyl, $C_1$-$C_4$-Alkoxycarbonyl, Benzyloxycarbonyl, oder eine Aminogruppe -$NR^bR^c$, worin

$R^b$ Wasserstoff, eine $C_1$-$C_4$-Alkylgruppe, eine $C_3$-$C_6$-Alkenylgruppe oder eine $C_3$-$C_6$-Alkinylgruppe und

$R^c$ Wasserstoff, eine $C_1$-$C_4$-Alkylgruppe, eine $C_3$-$C_6$-Alkenylgruppe, eine $C_3$-$C_6$-Alkinylgruppe, eine $C_1$-$C_6$-Acylgruppe oder eine Benzoylgruppe, die unsubstituiert sind oder ihrerseits ein bis drei der folgenden Reste tragen: Nitro, Cyano, Halogen, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy und/oder $C_1$-$C_4$-Alkoxycarbonyl bedeuten;

sowie ihre landwirtschaftlich nutzbaren Salze und Ester mit $C_1$-$C_{10}$-Carbonsäuren oder anorganischen Säuren.

2. Herbizides Mittel, enthaltend einen flüssigen oder festen Trägerstoff und eine herbizid wirksame Menge mindestens eines Cyclohexenonoximethers I gemäß Anspruch 1.

3. Verfahren zur Bekämpfung von unerwünschtem Pflanzenwuchs, dadurch gekennzeichnet, daß man eine herbizid wirksame Menge eines Cyclohexenonoximethers I gemäß Anspruch 1 auf Pflanzen, deren Lebensraum oder auf Saatgut einwirken läßt.

**Patentansprüche für folgenden Vertragsstaat : ES**

1. Herbizides Mittel, enthaltend einen flüssigen oder festen Trägerstoff und eine herbizid wirksame Menge mindestens eines Cyclohexenonoximethers I der allgemeinen Formel I

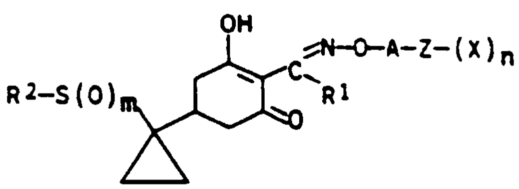

I

in der die Variablen folgende Bedeutung haben:
n 0 bis 5;
m 0 bis 2;
$R^1$ eine $C_1$-$C_6$-Alkylgruppe;
$R^2$ eine $C_1$-$C_4$-Alkylgruppe oder die Benzylgruppe;
A eine $C_1$-$C_6$-Alkylen-, eine $C_3$-$C_6$-Alkenylen- oder eine $C_3$-$C_6$-Alkinylengruppe, wobei diese Gruppen ein bis drei $C_1$-$C_3$-Alkylgruppen und/oder Halogenatome tragen können,
eine gewünschtenfalls mit ein bis drei $C_1$-$C_3$-Alkylgruppen substituierte $C_3$-$C_6$-Alkylen- oder $C_4$-$C_6$-Alkenylengruppe, in der eine Methylengruppe durch ein Sauerstoff-, ein Schwefelatom, eine Sulfoxid-, eine Sulfongruppe oder eine Gruppe -$N(R^a)$-, worin

$R^a$ Wasserstoff, eine $C_1$-$C_4$-Alkylgruppe, eine $C_3$-$C_6$-Alkenylgruppe oder eine $C_3$-$C_6$-Alkinylgruppe bedeutet, ersetzt ist;

Z Phenyl, ein 5-gliedriger Heteroaromat mit ein bis drei Stickstoffatomen und/oder einem Sauerstoff- oder Schwefelatom, ein 6-gliedriger Heteroaromat mit ein bis vier Stickstoffatomen;
X Nitro, Cyano, Halogen, $C_1$-$C_4$-Alkyl, partiell oder vollständig halogeniertes $C_1$-$C_4$-Alkyl, $C_1$-$C_4$-Alkoxy, $C_1$-$C_4$-Alkylthio, partiell oder vollständig halogeniertes $C_1$-$C_4$-Alkoxy, Carboxyl, $C_1$-$C_4$-Alkoxycarbonyl, Benzyloxycarbonyl, Phenyl, Pyridyl, wobei die aromatischen Reste unsubstituiert sind oder ein bis drei der folgenden Substituenten tragen: Nitro, Cyano, Halogen, $C_1$-$C_4$-Alkyl, partiell oder vollständig halogeniertes $C_1$-$C_4$-Alkyl, $C_1$-$C_4$-Alkoxy, $C_1$-$C_4$-Alkylthio, partiell oder vollständig halogeniertes $C_1$-$C_4$-Alkoxy, Carboxyl, $C_1$-$C_4$-Alkoxycarbonyl, Benzyloxycarbonyl, oder eine Aminogruppe -$NR^bR^c$, worin

$R^b$ Wasserstoff, eine $C_1$-$C_4$-Alkylgruppe, eine $C_3$-$C_6$-Alkenylgruppe oder eine $C_3$-$C_6$-Alkinylgrup-

pe und

$R^c$ Wasserstoff, eine $C_1$-$C_4$-Alkylgruppe, eine $C_3$-$C_6$-Alkenylgruppe, eine $C_3$-$C_6$-Alkinylgruppe, eine $C_1$-$C_6$-Acylgruppe oder eine Benzoylgruppe, die unsubstituiert sind oder ihrerseits ein bis drei der folgenden Reste tragen: Nitro, Cyano, Halogen, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy und/oder $C_1$-$C_4$-Alkoxycarbonyl bedeuten;

oder eines landwirtschaftlich nutzbaren Salzes von I oder eines Esters von I mit einer $C_1$-$C_{10}$-Carbonsäure oder einer anorganischen Säure.

**2.** Verfahren zur Bekämpfung von unerwünschtem Pflanzenwuchs, dadurch gekennzeichnet, daß man eine herbizid wirksame Menge eines Cyclohexenonoximethers I gemäß Anspruch 1 auf Pflanzen, deren Lebensraum oder auf Saatgut einwirken läßt.

**Claims**

**Claims for the following Contracting States : AT, BE, CH, DE, FR, GB, IT, LI, NL**

**1.** A cyclohexenone oxime ether of the formula I

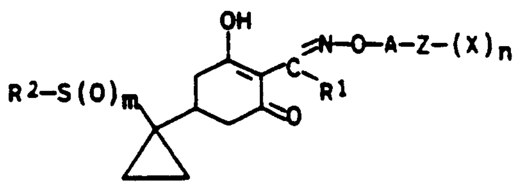

I

where

n is from 0 to 5;

m is from 0 to 2;

$R^1$ is $C_1$-$C_6$-alkyl;

$R^2$ is $C_1$-$C_4$-alkyl or benzyl;

A is $C_1$-$C_6$-alkylene, $C_3$-$C_6$-alkenylene or $C_3$-$C_6$-alkynylene, where these groups may carry from one to three $C_1$-$C_3$-alkyl groups and/or halogen atoms,

or a $C_3$-$C_6$-alkylene or $C_4$-$C_6$-alkenylene group which, if desired, is substituted by from one to three $C_1$-$C_3$-alkyl groups and in which one methylene group is replaced with an oxygen or sulfur atom, a sulfoxyl or sulfonyl group or a group -N($R^a$)-, where

$R^a$ is hydrogen, $C_1$-$C_4$-alkyl, $C_3$-$C_6$-alkenyl or $C_3$-$C_6$-alkynyl;

Z is phenyl, a 5-membered heteroaromatic radical having from one to three nitrogen atoms and/or one oxygen or sulfur atom or a 6-membered heteroaromatic radical having from one to four nitrogen atoms, and

X is nitro, cyano, halogen, $C_1$-$C_4$-alkyl, partially or completely halogenated $C_1$-$C_4$-alkyl, $C_1$-$C_4$-alkoxy, $C_1$-$C_4$-alkylthio, partially or completely halogenated $C_1$-$C_4$-alkoxy, carboxyl, $C_1$-$C_4$-alkoxycarbonyl, benzyloxycarbonyl, phenyl or pyridyl, where the aromatic radicals may be unsubstituted or carry from one to three of the following substituents: nitro, cyano, halogen, $C_1$-$C_4$-alkyl, partially or completely halogenated $C_1$-$C_4$-alkyl, $C_1$-$C_4$-alkoxy, $C_1$-$C_4$-alkylthio, partially or completely halogenated $C_1$-$C_4$-alkoxy, carboxyl, $C_1$-$C_4$-alkoxycarbonyl, benzyloxycarbonyl or an amino group -$NR^bR^c$, where

$R^b$ is hydrogen, $C_1$-$C_4$-alkyl, $C_3$-$C_6$-alkenyl or $C_3$-$C_6$-alkynyl and

$R^c$ is hydrogen, $C_1$-$C_4$-alkyl, $C_3$-$C_6$-alkenyl, $C_3$-$C_6$-alkynyl, $C_1$-$C_6$-acyl or benzoyl which may be unsubstituted or in its turn carry from one to three of the following radicals: nitro, cyano, halogen, $C_1$-$C_4$-alkyl, $C_1$-$C_4$-alkoxy, $C_1$-$C_4$-alkylthio, $C_1$-$C_4$-haloalkyl, $C_1$-$C_4$-haloalkoxy and/or $C_1$-$C_4$-alkoxycarbonyl,

and its agriculturally useful salts and esters with $C_1$-$C_{10}$-carboxylic acids or inorganic acids.

**2.** A herbicide containing a liquid or solid carrier and a herbicidal amount of one or more cyclohexenone oxime ethers I as claimed in claim 1.

**3.** A method for controlling undesirable plant growth, wherein a herbicidal amount of a cyclohexenone oxime ether I as claimed in claim 1 is allowed to act on plants or their habitat or on seed.

**Claims for the following Contracting State : ES**

**1.** A herbicide containing a liquid or solid carrier and a herbicidal amount of one or more cyclohexenone oxime ethers I of the formula I

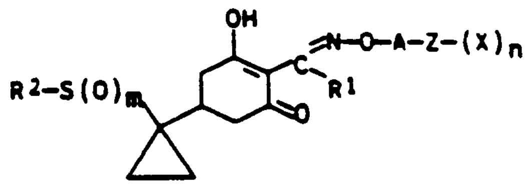

I

where
n is from 0 to 5;
m is from 0 to 2;
$R^1$ is $C_1$-$C_6$-alkyl;
$R^2$ is $C_1$-$C_4$-alkyl or benzyl;
A is $C_1$-$C_6$-alkylene, $C_3$-$C_6$-alkenylene or $C_3$-$C_6$-alkynylene, where these groups may carry from one to three $C_1$-$C_3$-alkyl groups and/or halogen atoms,
or a $C_3$-$C_6$-alkylene or $C_4$-$C_6$-alkenylene group which, if desired, is substituted by from one to three $C_1$-$C_3$-alkyl groups and in which one methylene group is replaced with an oxygen or sulfur atom, a sulfoxyl or sulfonyl group or a group -N($R^a$)-, where
$R^a$ is hydrogen, $C_1$-$C_4$-alkyl, $C_3$-$C_6$-alkenyl or $C_3$-$C_6$-alkynyl;
Z is phenyl, a 5-membered heteroaromatic radical having from one to three nitrogen atoms and/or one oxygen or sulfur atom or a 6-membered heteroaromatic radical having from one to four nitrogen atoms, and
X is nitro, cyano, halogen, $C_1$-$C_4$-alkyl, partially or completely halogenated $C_1$-$C_4$-alkyl, $C_1$-$C_4$-alkoxy, $C_1$-$C_4$-alkylthio, partially or completely halogenated $C_1$-$C_4$-alkoxy, carboxyl, $C_1$-$C_4$-alkoxycarbonyl, benzyloxycarbonyl, phenyl or pyridyl, where the aromatic radicals may be unsubstituted or carry from one to three of the following substituents: nitro, cyano, halogen, $C_1$-$C_4$-alkyl, partially or completely halogenated $C_1$-$C_4$-alkyl, $C_1$-$C_4$-alkoxy, $C_1$-$C_4$-alkylthio, partially or completely halogenated $C_1$-$C_4$-alkoxy, carboxyl, $C_1$-$C_4$-alkoxycarbonyl, benzyloxycarbonyl or an amino group -$NR^bR^c$, where
$R^b$ is hydrogen, $C_1$-$C_4$-alkyl, $C_3$-$C_6$-alkenyl or $C_3$-$C_6$-alkynyl and
$R^c$ is hydrogen, $C_1$-$C_4$-alkyl, $C_3$-$C_6$-alkenyl, $C_3$-$C_6$-alkynyl, $C_1$-$C_6$-acyl or benzoyl which may be unsubstituted or in its turn carry from one to three of the following radicals: nitro, cyano, halogen, $C_1$-$C_4$-alkyl, $C_1$-$C_4$-alkoxy, $C_1$-$C_4$-alkylthio, $C_1$-$C_4$-haloalkyl, $C_1$-$C_4$-haloalkoxy and/or $C_1$-$C_4$-alkoxycarbonyl,
or of an agriculturally useful salt of I or of an ester of I with a $C_1$-$C_{10}$-carboxylic acid or an inorganic acid.

**2.** A method for controlling undesirable plant growth, wherein a herbicidal amount of a cyclohexenone oxime ether I as claimed in claim 1 is allowed to act on plants or their habitat or on seed.

**Revendications**
**Revendications pour les Etats contractants suivants : AT, BE, CH, DE, FR, GB, IT, LI, NL**

**1.** Cyclohexénonoximéther de formule générale I

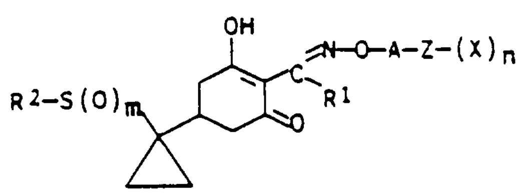

I

dans laquelle les variables ont la signification suivante :
n 0 à 5 ;
m 0 à 2 ;

$R^1$ un groupe alkyle en C1-C6 ;
$R^2$ un groupe alkyle en C1-C4 ou le groupe benzyle ;
A un groupe alkylène en C1-C6, un groupe alcénylène en C3-C6 ou un groupe alkinylène en C3-C6, ces groupes pouvant porter un à trois groupes alkyle en C1-C3 et/ou des atomes d'halogène,
un groupe alkylène en C3-C6 ou alcénylène en C4-C6 éventuellement substitué avec un à trois groupes alkyle en C1-C3 et dans lequel un groupe méthylène est remplacé par un atome d'oxygène, un atome de soufre, un groupe sulfoxyde, un groupe sulfone ou un groupe -N($R^a$)- où
$R^a$ représente hydrogène, un groupe alkyle en C1-C4, un groupe alcényle en C3-C6 ou un groupe alkinyle en C3-C6 ;
Z phényle, un hétéroaromate à 5 chaînons avec un à trois atomes d'azote et/ou un atome d'oxygène ou de soufre, un hétéroaromate à 6 chaînons avec un à quatre atomes d'azote ;
X représente nitro, cyano, halogène, alkyle en C1-C4, alkyle en C1-C4 partiellement ou totalement halogéné, alcoxy en C1-C4, alkylthio en C1-C4, alcoxy en C1-C4 partiellement ou totalement halogéné, carboxyle, alcoxycarbonyle en C1-C4, benzyloxycarbonyle, phényle, pyridyle, les restes aromatiques étant non substitués ou portent un à trois des substituants suivants : cyano, nitro, halogène, alkyle en C1-C4, alkyle en C1-C4 partiellement ou totalement halogéné, alcoxy en C1-C4, alkylthio en C1-C4, alcoxy en C1-C4 partiellement ou totalement halogéné, carboxyle, alkoxycarbonyle en C1-C4, benzyloxycarbonyle ou un groupe amino -N$R^b$$R^c$ où
$R^b$ représente hydrogène, un groupe alkyle en C1-C4, un groupe alcényle en C3-C6 ou un groupe alkinyle en C3-C6 et
$R^c$ représente hydrogène, un groupe alkyle en C1-C4, un groupe alcényle en C3-C6, un groupe alkinyle en C3-C6, un groupe acyle en C1-C6 ou un groupe benzyle, qui sont non substitués ou à leur tour portent un à trois des restes suivants : nitro, cyano, halogène, alkyle en C1-C4, alcoxy en C1-C4, alkylthio en C1-C4, halogénalkyle en C1-C4, halogénalcoxy en C1-C4 et/ou alcoxycarbonyle en C1-C4 ;
ainsi que leurs sels et esters avec des acides carboxyliques en C1-C10 ou des acides minéraux utilisables en agriculture.

2. Agent herbicide, contenant un support liquide ou solide et une quantité à efficacité herbicide d'au moins un cyclohexénonoximéther I selon la revendication 1.

3. Procédé pour lutter contre la croissance de plantes indésirables, caractérisé par le fait que l'on fait agir une quantité à efficacité herbicide d'un cyclohexénonoximéther I selon la revendication 1 sur les plantes, leur biotope ou les semences.

**Revendications pour l'Etat contractant suivant : ES**

1. Agent herbicide, contenant un support liquide ou solide et une quantité à efficacité herbicide d'au moins un cyclohexénonoximéther I de formule générale I

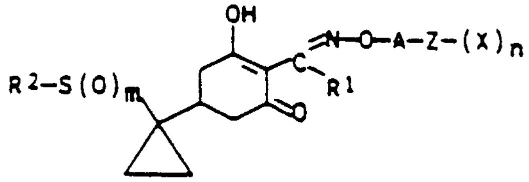

I

dans laquelle les variables ont la signification suivante :
n 0 à 5 ;
m 0 à 2 ;
$R^1$ un groupe alkyle en C1-C6 ;
$R^2$ un groupe alkyle en C1-C4 ou le groupe benzyle ;
A un groupe alkylène en C1-C6, un groupe alcénylène en C3-C6 ou un groupe alkinylène en C3-C6, ces groupes pouvant porter un à trois groupes alkyle en C1-C3 et/ou des atomes d'halogène,
un groupe alkylène en C3-C6 ou alcénylène en C4-C6 éventuellement substitué avec un à trois groupes alkyle en C1-C3 et dans lequel un groupe méthylène est remplacé par un atome d'oxygène, un atome de soufre, un groupe sulfoxyde, un groupe sulfone ou un groupe -N($R^a$)- où
$R^a$ représente hydrogène, un groupe alkyle en C1-C4, un groupe alcényle en C3-C6 ou un groupe

alkinyle en C3-C6 ;
Z phényle, un hétéroaromate à 5 chaînons avec un à trois atomes d'azote et/ou un atome d'oxygène ou de soufre, un hétéroaromate à 6 chaînons avec un à quatre atomes d'azote ;
X représente nitro, cyano, halogène, alkyle en C1-C4, alkyle en C1-C4 partiellement ou totalement halogéné, alcoxy en C1-C4, alkylthio en C1-C4, alcoxy en C1-C4 partiellement ou totalement halogéné, carboxyle, alcoxycarbonyle en C1-C4, benzyloxycarbonyle, phényle, pyridyle, les restes aromatiques étant non substitués ou portent un à trois des substituants suivants : cyano, nitro, halogène, alkyle en C1-C4, alkyle en C1-C4 partiellement ou totalement halogéné, alcoxy en C1-C4, alkylthio en C1-C4, alcoxy en C1-C4 partiellement ou totalement halogéné, carboxyle, alkoxycarbonyle en C1-C4, benzyloxycarbonyle ou un groupe amino $-NR^bR^c$ où
$R^b$ représente hydrogène, un groupe alkyle en C1-C4, un groupe alcényle en C3-C6 ou un groupe alkinyle en C3-C6 et
$R^c$ représente hydrogène, un groupe alkyle en C1-C4, un groupe alcényle en C3-C6, un groupe alkinyle en C3-C6, un groupe acyle en C1-C6 ou un groupe benzyle, qui sont non substitués ou à leur tour portent un à trois des restes suivants : nitro, cyano, halogène, alkyle en C1-C4, alcoxy en C1-C4, alkylthio en C1-C4, halogénalkyle en C1-C4, halogénalcoxy en C1-C4 et/ou alcoxycarbonyle en C1-C4 ;
ou un sel de I en agriculture ou un ester de I avec un acide carboxylique en C1-C10 ou un acide minéral.

**2.** Procédé pour lutter contre la croissance de plantes indésirables, caractérisé par le fait que l'on fait agir une quantité à efficacité herbicide d'un cyclohexénonoximéther I selon la revendication 1 sur les plantes, leur biotope ou les semences.